(19) Europäisches Patentamt
European Patent Office
Office européen des brevets

(11) Publication number: **0 078 668**
**A1**

(12) **EUROPEAN PATENT APPLICATION**

(21) Application number: **82305730.2**

(22) Date of filing: **28.10.82**

(51) Int. Cl.³: **C 07 D 295/14**, C 07 F 7/08

(30) Priority: **29.10.81 GB 8132671**

(43) Date of publication of application: **11.05.83**
**Bulletin 83/19**

(84) Designated Contracting States: **AT BE CH DE FR GB IT LI LU NL SE**

(71) Applicant: **GLAXO GROUP LIMITED, Clarges House 6-12 Clarges Street, London W1Y 8DH (GB)**

(72) Inventor: **Waterhouse, Ian, 50 Melbourn Road, Royston Hertfordshire (GB)**
Inventor: **Marshall, David R., 17 Valley Close, Hertford Hertfordshire (GB)**
Inventor: **Collington, Eric W., 103 Warren Way Digswell, Welwyn Hertfordshire (GB)**
Inventor: **Wallis, Christopher J., The White House Noons Folly Newmarket Road, Royston Hertfordshire (GB)**
Inventor: **Hallett, Peter, 46 Walnut Tree Close Bassingbourn, Royston Hertfordshire (GB)**

(74) Representative: **Skailes, Humphrey John et al, Frank B. Dehn & Co. Imperial House 15-19 Kingsway, London WC2B 6UZ (GB)**

(54) **Preparation of aminocyclopentanealkenoic acids.**

(57) A process is described for the preparation of compounds of the formula (1)

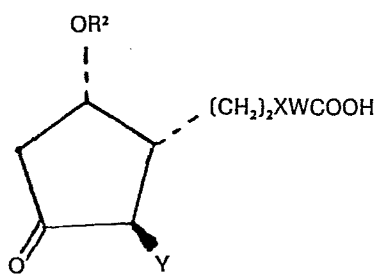

(1)

(and their salts and hydrates) in which:
W is alkylene
X is cis or trans -CH=CH-;
Y is a saturated heterocyclic amino group having 5-8 ring members; and
$R^2$ is $C_{3-6}$ alkenyl (optionally substituted), $C_{1-12}$ alkyl, or substituted or unsubstituted phenylalkyl, thienylalkyl, furanylalkyl, biphenylalkyl or naphthylalkyl.
The process comprises hydrolysing an ester of the acid (1).

EP 0 078 668 A1

Preparation of aminocyclopentanealkenoic acids

This invention concerns the preparation of aminocyclopentanealkenoic acids, and is particularly concerned with the preparation of compounds of the general formula (1)

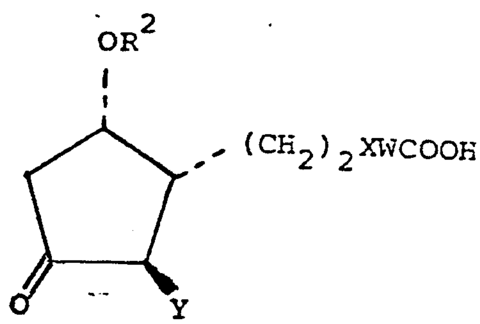

(1)

(1)

wherein X is cis or trans -CH=CH-;
W is straight or branched $C_{1-7}$ alkylene;
Y represents a saturated heterocyclic amino group which has 5-8 ring members and (a) optionally contains in the ring -O-, -S-, $-SO_2-$, $-NR^4-$ (where $R^4$ is a hydrogen atom, $C_{1-7}$ alkyl or aralkyl having a $C_{1-4}$ alkyl portion); and/or (b) is optionally substituted by one or more $C_{1-4}$ alkyl groups;

$R^2$ is (i) $C_{3-6}$ alkenyl, optionally substituted by phenyl (the phenyl being optionally substituted by $C_{1-4}$ alkyl, $C_{1-4}$ alkoxy, halogen, $C_{5-7}$ cycloalkyl or phenyl ($C_{1-4}$) alkyl), biphenyl (optionally substituted by $C_{1-4}$ alkyl, $C_{1-4}$ alkoxy or halogen), or naphthyl; (ii) $C_{1-12}$ alkyl; (iii) $C_{1-5}$ alkyl substituted by (a) phenyl [optionally substituted by halogen, hydroxy, $C_{1-6}$ alkyl, $C_{1-6}$ alkoxy, $C_{1-4}$ hydroxyalkoxy, trifluoromethyl, cyano, aryloxy (e.g. phenoxy), $C_{5-7}$ cycloalkyl, aralkoxy (e.g. benzyloxy), dimethylaminomethyl, carboxamido ($-CONH_2$), thiocarboxamido ($-CSNH_2$), $C_{1-4}$ alkanoyl, $-NR^5R^6$ (where $R^5$ and $R^6$ are the same or different

and are each a hydrogen atom or $C_{1-4}$ alkyl, or where $-NR^5R^6$ is a saturated heterocyclic amino group as defined above for Y), $C_{1-3}$ alkylthio, $C_{1-3}$ alkylsulphinyl, $C_{1-3}$ alkylsulphonyl, phenylalkyl having a $C_{1-3}$ alkyl portion, aminosulphonyl, $C_{1-3}$ alkanoylaminosulphonyl, phenylsulphonyl (the phenyl portion being optionally substituted by $C_{1-3}$ alkyl or $C_{1-3}$ alkoxy), nitro, or thienyl], (b) thienyl or furanyl [the thienyl and furanyl groups being optionally substituted by $C_{1-6}$ alkyl, $C_{1-6}$ alkoxy, aryl (e.g. phenyl) or phenyl ($C_{1-3}$) alkyl or phenyl ($C_{1-3}$) alkoxy (the aryl or phenyl group in each case being optionally substituted by $C_{1-3}$ alkyl, $C_{1-3}$ alkoxy or halogen), aryloxy (e.g. phenoxy), $C_{5-7}$ cycloalkyl, halogen, nitro or thienyl], (c) biphenyl (optionally substituted by phenyl or one or two $C_{1-4}$ alkyl, $C_{1-4}$ alkoxy or halogen substituents), or (d) naphthyl (optionally substituted by $C_{1-4}$ alkyl, $C_{1-4}$ alkoxy or halogen);

and the physiologically acceptable salts and the solvates (e.g. hydrates) thereof.

The structural formulae herein are to be understood to include the enantiomers of each of the compounds concerned as well as mixtures of the enantiomers, including racemates, even though the precise structure as set out only relates to one enantiomer.

These compounds have shown endoperoxide and thromboxane antagonist activity and are therefore of interest in the treatment of asthma and cardiovascular diseases.

This invention provides a process for the preparation of a compound of formula (1) or a salt or solvate thereof, which comprises hydrolysing an ester of formula (2)

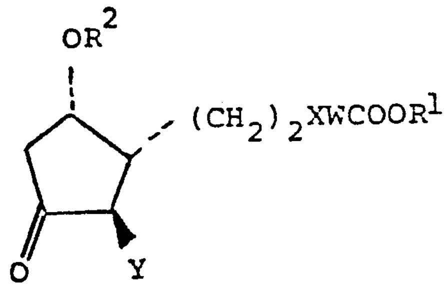

(2)

in which $R^1$ is:

(a) $-CR^7R^8R^9$ in which $R^7$ and $R^8$ are each phenyl (optionally substituted by $C_{1-4}$ alkyl, $C_{1-4}$ alkoxy, di-$(C_{1-4})$alkylamino, nitro or halogen) and $R^9$ is a hydrogen atom or a substituted or unsubstituted phenyl group as defined for $R^7$ and $R^8$;

(b) $-CH_2BR^{10}$ where B is -O- or -S- and $R^{10}$ is $C_{1-4}$ alkyl;

(c) $-CH_2OCOR^{11}$ where $R^{11}$ is $C_{1-4}$ alkyl or methoxy;

(d) $-CH(R^{12})OCOCH_3$ where $R^{12}$ is methyl or phenyl;

(e) tetrahydro-5-oxo-2-furanyl;

(f) $-CH_2CH_2SiR^{13}_3$ where $R^{13}$ is $C_{1-6}$ alkyl, e.g. methyl;

(g) $-CH_2CCl_3$; or

(h) $-SiR^{14}R^{15}R^{16}$ where $R^{14}$, $R^{15}$ and $R^{16}$ are aryl (e.g. phenyl) or $C_{1-6}$ alkyl, at least one of $R^{14}$, $R^{15}$ and $R^{16}$ being aryl.

$R^1$ is preferably a group (a), for example triphenylmethyl (in which the phenyl groups are optionally substituted by methyl, methoxy or nitro) or diphenylmethyl (in which the phenyl groups are optionally substituted by halogen, methyl, methoxy or dimethylamino). More preferably, $R^1$ is triphenylmethyl.

The hydrolysis of the esters of formula (2) can in general be effected under neutral or mildly acidic or basic conditions, optionally in an organic or aqueous

organic solvent and at any suitable temperature, conveniently -5 to 25°C, preferably at room temperatures.

Thus when $R^1$ is a group of the type (a), (b), (c), (d), (e), (f), or (h), hydrolysis may be effected in the presence of a strong acid, preferably an organic acid such as trifluoroacetic acid.

Suitable solvents for such hydrolyses include tetrahydrofuran, ether, aqueous ether and $CH_2Cl_2$. The reaction may be performed at room temperature.

Alternatively, esters in which $R^1$ is a group (h) can be hydrolysed with an acid such as acetic acid in a suitable solvent (e.g. aqueous tetrahydrofuran). This reaction may be performed at room temperature.

When $R^1$ is a group of type (b), hydrolysis may also be effected under neutral conditions in the presence of $HgCl_2$ (for example using aqueous $CH_3CN$ as solvent). These esters may also be hydrolysed in the presence of $AgNO_3$, for example using buffered aqueous tetrahydrofuran, dioxan or dimethoxyethane as solvent. The reaction is preferably effected at room temperature.

Esters in which $R^1$ is $-CH_2CCl_3$ may be hydrolysed by treatment with zinc, for example at a pH of 4.2-7.2. Tetrahydrofuran, dioxan and dimethoxyethane are suitable solvents. The reation is preferably effected at room temperature.

Esters in which $R^1$ is a group (h) may also be hydrolysed by a quaternary ammonium fluoride (e.g. $(t\text{-}But)_4NF$) or HF, for example using tetrahydrofuran or $CH_3CN$ as the reaction solvent. This reaction may be performed at room temperature.

Esters in which $R^1$ is a group of the type (c), (d) or (e) may also be hydrolysed under basic conditions, for example in the presence of an inorganic base such as $Na_2HPO_4$. The solvent may for example be an aqueous

alcohol (e.g. methanol) and the reaction may be performed at room temperature.

The acid of formula (1) produced is conveniently isolated in the form of a salt, for example a salt with an organic base, such as piperidine. If desired, the free acid may be liberated from the salt, and converted into another salt if required. The salt initially isolated may also be converted directly into another salt without liberation of the acid, by exchange of cation.

Thus, salts of inorganic bases may be prepared by adding the base to a solution of the acid of formula (1) in an aqueous organic solvent. Certain salts may also be prepared by exchange of cation; for example, calcium salt may be prepared by addition of a calcium salt (e.g. the chloride or acetate) to a solution of a salt of a compound of formula (1), e.g. an amine or alkali metal salt.

In a further aspect of the invention the esters of formula (2) are prepared by oxidising a corresponding hydroxy compound of formula (3)

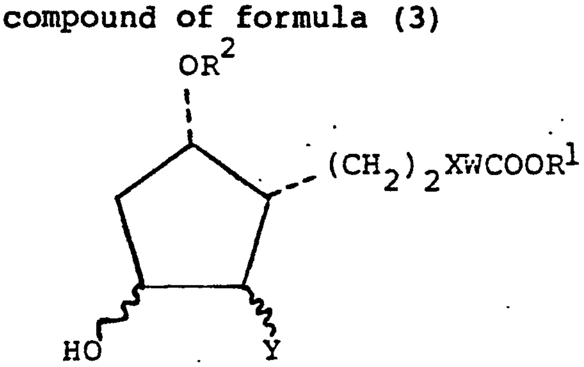

(3)

(other than compounds in which Y is in the α-configuration and the ring hydroxy group is in the β-configuration).

Suitable methods of oxidation include using a $Cr^{vi}$ oxidising reagent in a suitable solvent, e.g. chromic acid in acetone (e.g. Jones reagent, preferably used in the presence of a diatomaceous silica such as Celite) or $CrO_3$ in pyridine. These reagents are for example used at temperatures of -20°C to room temperature.

Other important methods include using an activated sulphur reagent, e.g. (i) N-chlorosuccinimide-dimethylsulphide complex in a suitable solvent (e.g. toluene or dichloromethane) at temperatures of for example -25 to 25°C, preferably at 0-5°, (ii) a dialkyl sulphoxide (e.g. dimethylsulphoxide) activated by a suitable electrophilic reagent (such as oxalyl chloride, acetyl bromide or thionyl chloride) in a suitable solvent (e.g. toluene or dichloromethane), e.g. at -70 to -20°C; dicyclohexylcarbodiimide can also be used as the electrophilic reagent (preferably in the presence of $CF_3COOH$ or its pyridinium salt) at for example -10°C to room temperature, using the same solvents, or (iii) pyridine-$SO_3$ complex in dimethylsulphoxide, preferably at 0°C to room temperature.

The choice of oxidation method will depend on the nature of $R^1$.

When Y is in the α-configuration conditions should be chosen to effect epimerisation after oxidation, for example by using a $Cr^{vi}$ oxidising agent.

Any hydroxy or amino group present in the starting material and required in the end product should be suitable protected in this reaction.

The compounds of formula (3) may be prepared by esterification of the corresponding carboxylic acid, i.e. a compound of formula (3) in which $R^1$ represents a hydrogen atom. Conventional esterification methods may be used.

For example, compounds of formula (3) in which $R^1$ is a group of the type (f) or (g) may be prepared by treating a reactive derivative of the corresponding carboxylic acid with an appropriate alcohol $R^1OH$. The reactions may for example be carried out at -10°C to room temperature using a solvent such as acetone.

The reactive derivative is conveniently a mixed anhydride of the acid, formed for example by treatment of the acid with a chloroformate in the presence of

a suitable base, e.g. triethylamine at -12°C.

The chloroformate may for example be a $C_{1-6}$ alkyl (e.g. iso-butyl), aryl (e.g. phenyl) or aralkyl (e.g. benzyl) chloroformate.

Again for example, compounds of formula (3) in which $R^1$ is a group of the type (a), (b), (c), (d), (e), or (h) may be prepared by reacting the corresponding carboxylic acid with an appropriate halide $R^{17}$-Hal, where Hal represents halogen and $R^{17}$ is as just defined for $R^1$. The reaction is carried out in the presence of a suitable base, e.g. potassium t-butoxide or a sterically hindered amine such as triethylamine, N,N-diisopropylethylamine, or dicyclohexylamine in a suitable solvent (such as acetonitrile, dimethylsulphoxide, dimethylformamide or $CH_2Cl_2$) for example at a temperature from 0°C to room temperature.

In another example, compounds of formula (3) in which $R^1$ is a group of the type (a) where $R^9$ is a hydrogen atom may be prepared by reacting the corresponding carboxylic acid with an appropriate diphenyldiazomethane in a solvent such as benzene at e.g. room temperature.

The parent carboxylic acids required for the preparation of the esters of formula (3) may be prepared as described in British Patent Specification 2075503A.

The process of the invention is particularly applicable to the preparation of compounds of formula (1) as defined below.

The alkyl groups referred to above in the definition of the compounds of formula (1) may be straight or branched.

W may for example contain 1-5 carbon atoms in a straight or branched chain, and is preferably $-CH_2CH_2-$.

The compounds of formula (1) are capable of salt formation with bases and the compounds are preferably used in the form of such salts. Examples of suitable salts are alkali metal (e.g. sodium and potassium),

alkaline earth metal (e.g. calcium or magnesium), ammonium, substituted ammonium (e.g. tromethamine or dimethylamino-ethanol), piperazine, N,N-dimethylpiperazine, morpholine, piperidine and tertiary amino (e.g. triethylamine) salts. Inorganic salts are preferred.

X is preferably a <u>cis</u> -CH=CH- group.

The heterocyclic amino group Y may for example have a 5, 6 or 7-membered ring, e.g. pyrrolidino, piperidino, morpholino, piperazino, thiomorpholino, 1,1-dioxothiomorpholino, homomorpholino and hexamethyleneimino. Examples of the optional substituents which may be present on a second nitrogen atom in the ring are methyl, ethyl and benzyl. The carbon atoms of the heterocyclic rings may for example be substituted by methyl or ethyl. Y is preferably piperidino, morpholino, homomorpholino, thiomorpholino or 1,1-dioxothiomorpholino, and compounds in which Y is a morpholino or piperidino group are particularly preferred.

The amino group Y enables the compounds to form salts with organic acids, e.g. maleates.

$R^2$ may for example be $C_{5-10}$ alkyl (e.g. pentyl or decyl); $C_{3-5}$ alkenyl (e.g. allyl, optionally substituted by phenyl); or $C_{1-5}$ alkyl (e.g. methyl or propyl) substituted by phenyl [optionally substituted by a $C_{1-4}$ alkyl (e.g. tert.butyl), $C_{5-7}$ cycloalkyl (e.g. cyclohexyl), $C_{1-3}$ alkylthio (e.g. methylthio), phenyl ($C_{1-3}$) alkyl (e.g. benzyl) or thienyl], furanyl or thienyl (optionally substituted by a phenyl group), biphenyl [optionally substituted by $C_{1-3}$ alkyl (e.g. methyl), $C_{1-3}$ alkoxy (e.g. methoxy), halogen (e.g. chlorine) or phenyl], or naphthyl.

$R^2$ is preferably a phenylalkyl group in which the alkyl portion contains 1-3 carbon atoms and the phenyl is substituted with one of the following groups: $C_{1-3}$ alkylthio, thienyl or phenyl optionally substituted by $C_{1-3}$ alkyl, $C_{1-3}$ alkoxy, halogen or phenyl; or is thienylalkyl in which the alkyl portion contains

1-3 carbon atoms and the thienyl group is substituted by a phenyl group; or cinnamyl.

Particularly preferred $R^2$ groups are phenylalkyl groups in which the alkyl portion is a $C_{1-3}$ alkylene chain and the phenyl group carries a phenyl substituent, preferably in the para-position (which phenyl substituent is optionally substituted by a $C_{1-3}$ alkyl, $C_{1-3}$ alkoxy or halogen, this additional substituent preferably being in the meta or more particularly the para-position); or thienylmethyl group (particularly a 4-thienylmethyl group) substituted by a phenyl group, which substituent is preferably in the 2-position; or cinnamyl.

Especially important $R^2$ groups are benzyl groups substituted (preferably in the para-position) by phenyl, 4-methoxyphenyl or 4-methylphenyl.

A particularly preferred group of compounds has the formula (1) in which:

X is cis $-CH=CH-$,

W is $-CH_2CH_2-$

Y is morpholino or piperidino, and

$R^2$ is phenyl $(C_{1-3})$ alkyl in which the phenyl group is substituted by phenyl (which phenyl substituent is optionally substituted by $C_{1-3}$ alkyl, $C_{1-3}$ alkoxy or halogen); phenylthienylmethyl; or cinnamyl,

and the physiologically acceptable salts and solvates (e.g. hydrates) thereof.

Particularly important compounds in this latter group are those in which Y is morpholino and $R^2$ is 1,1'-biphenylmethyl; 1,1'-biphenylmethyl substituted in the para-position by methyl, methoxy or chloro or in the meta-position by methoxy; 1,1'-biphenylpropyl; 2-phenyl-thien-4-yl-methyl; or cinnamyl; and those in which Y is piperidino and $R^2$ is 1,1'-biphenylmethyl or 4'-methoxy-1-1'-biphenylmethyl. Especially important are:

[1α(Z),2β,5α]-(±)-7-[5[[(1,1'-biphenyl)-4-yl]methoxy]-2-(4-morpholinyl)-3-oxocyclopentyl]-4-heptenoic acid; and

[1R-[1α(Z),2β,5α]]-(-)-7-[5-[[(1,1'-biphenyl)-4-yl]methoxy]-2-(4-morpholinyl)-3-oxocyclopentyl]-4-heptenoic acid; and the hydrates and salts thereof, particularly the calcium, piperidine, piperazine and N,N-dimethylpiperazine salts. The calcium salts are particularly important.

In general, compounds of formula (1) in which the carbon atom carrying the $-(CH_2)_2XWCOOH$ group is in the R-configuration (and mixtures containing this isomer) are preferred.

The following examples illustrate the invention. Temperatures are in °C. The following abbreviations are used:

TLC - thin layer chromatography using $SiO_2$; PE - petroleum ether (boiling at 40-60°); THF - tetrahydrofuran; EA - ethyl acetate; HOAc - acetic acid; DMSO - dimethylsulphoxide; DMF-dimethylformamide. Chromatography was carried out using silica gel unless otherwise stated. 'Dried' refers to drying with $MgSO_4$. 'Hyflo' is a filtration aid.

The proportion of the following intermediates is described in British Patent Specification 2075503A

Intermediate 1

[1α(Z),2β,3α,5α]-(±)-7-[5-[[(1,1'-Biphenyl)-4-yl]methoxy]-3-hydroxy-2-(4-morpholinyl)cyclopentyl]-4-heptenoic acid.

Intermediate 2

[1R-[1α(Z),2β,3α,5α]]-(+)-7-[5-[[(1,1'-Biphenyl)-4-yl]methoxy]-3-hydroxy-2-(4-morpholinyl)cyclopentyl]-4-heptenoic acid.

Intermediate 3

[1α(Z),2β,5α]-(±)-7-[5-[[(1,1'-Biphenyl)-4-yl]methoxy]-2-(4-morpholinyl)-3-oxocyclopentyl]]-4-heptenoic acid.

Intermediate 4

[1R-[1α(Z),2β,5α]]-(-)-7-[5-[[(1,1'-Biphenyl)-4-yl]methoxy]-2-(4-morpholinyl)-3-oxocyclopentyl]-4-heptenoic acid.

Intermediate 5

[1α(Z),2β,5α]-(±)-Triphenylmethyl 7-[5-[[(1,1'-Biphenyl)-4-yl]methoxy]-2-(4-morpholinyl)-3-oxocyclopentyl]-4-heptenoate

Triphenylmethyl chloride (1.315g) was added at 2° to a solution of Intermediate 3 (1.5g) and triethylamine (0.88ml) in $CH_2Cl_2$ (9ml). After 1h at 2-4° the mixture was diluted with pH 6 phosphate buffer (75ml) and ether (75ml). The ether layer was washed with water (2x25ml), dried and evaporated. The residue was purified by chromatography using ether as eluent to give the title compound as a foam (1.843g).

Analysis Found: C,80.2; H,6.9; N,2.1;

$C_{48}H_{49}NO_5$ required: C,80.1; H,6.9; N,1.95%

Intermediate 6

[1R-[1α(Z),2β,5α]]-(-)-Triphenylmethyl 7-[5-[[(1,1'-Biphenyl)-4-yl]methoxy]-2-(4-morpholinyl)-3-oxocyclopentyl]-4-heptenoate

To a cooled (5-10°) stirred solution of Intermediate 2 methane sulphonate (1g) and triphenylmethyl chloride

(0.6g) in $CH_2Cl_2$ (5ml) was added triethylamine (0.6ml). The mixture was stirred at less than 10° for 0.5h when more triethylamine (1.7 ml) was added followed by a solution of pyridine - sulphur trioxide complex (1.1g) in DMSO (5ml). The cooling bath was removed and the mixture was stirred at ambient temperature for 1.5h. Water (30ml) was added and the mixture was extracted with ether (2x20ml). The combined extracts were washed with water (15ml), 1M citric acid (8ml) and water (10ml), dried and evaporated. The residue was purified by chromatography using 2:1 ether -PE as eluent to give the title compound as a foam (0.75g).

Analysis Found: C,80.0; H,6.7; N,1.8;

$C_{48}H_{49}NO_5$ requires: C,80.1; H,6.9; N,1.95%

$[\alpha]^{22}_{D} = -7.3°$ ($CHCl_3$)

Intermediate 7

[1α(Z),2β,5α]-(±)-Diphenylmethyl 7-[5-[[(1,1'-Biphenyl)-4-yl]methoxy]-2-(4-morpholinyl)-3-oxocyclopentyl]-4-heptenoate

A solution of Intermediate 3 (0.5g) and diphenyldiazomethane (0.6g) in benzene (15ml) was stirred at ambient temperature for 4h. The solvent was removed *in vacuo* and the residue was purified by chromatography using ether as eluent to give the title compound as an oil (0.484g).

Analysis Found: C,78.3; H,7.0 ; N,2.15;

$C_{42}H_{45}NO_5$ requires: C,78.35; H,7.05; N,2.1%

Intermediate 8

[1R-[1α(Z),2β,5α]]-(-)-(Acetyloxy)methyl 7-[5-((1,1'-Biphenyl)-4-yl]methoxy]-2-(4-morpholinyl)-3-oxocyclopentyl]-4-heptenoate

A solution of Intermediate 4 (0.436g) in acetone (6ml) containing triethylamine (0.3ml) and bromomethylacetate (0.3g) was stirred at 20° for 2h. The mixture was poured into pH 6.5 phosphate buffer (50ml) and extracted with ether (3x50ml). The combined extracts were dried and evaporated to give an oil. Purification

by chromatography using ether as eluent gave the title compound as an oil (0.351g).

Analysis Found: C,69.8; H,7.3; N,2.4;

$C_{32}H_{39}NO_7$ requires: C,69.9; H,7.15; N,2.55%

$[\alpha]_D^{21.5}$ = -8.8° ($CHCl_3$)

Intermediate 9

a) [1α(Z),2β,5α]-(±)-2,2,2-Trichloroethyl 7-[5-[[(1,1'-Biphenyl)-4-yl]methoxy]-2-(4-morpholinyl)-3-oxocyclopentyl]-4-heptenoate

Iso-butyl chloroformate (0.68ml) was added to a cooled (-12°) stirred solution of Intermediate 3 (0.8g) and triethylamine (0.92ml) in acetone (32ml) under nitrogen. After 0.5h 2,2,2-trichloroethanol (0.76ml) was added and 0.5h later the mixture was allowed to warm to 0°. After 3.5h the mixture was diluted with pH 6.5 phosphate buffer (60ml) and extracted with ether (3x65ml). The combined extracts were dried and evaporated and the residue was purified initially by chromatography using 1:1 ether - PE as eluent then by trituration with ether to give the title compound as a solid (0.22g) m.p. 86.5-88°.

b) [1α(Z),2β,5α]-(±)-(2-Trimethylsilylethyl) 7-[5-[[(1,1'-Biphenyl)-4-yl]methoxy]-2-(4-morpholinyl)-3-oxocyclopentyl]-4-heptenoate, m.p. 43.5-45° from Intermediate 3 and trimethylsilylethanol. Purification by chromatography using 1:2 ether - PE as eluent.

Intermediate 10

[1R-[1α(Z),2β,3α,5α]]-(+)-Methoxymethyl 7-[5-[[(1,1'-Biphenyl)-4-yl]-methoxy]-3-hydroxy-2-(4-morpholinyl)-cyclopentyl]-4-heptenoate

Chloromethylmethyl ether (0.16 ml) was added to a stirred solution of Intermediate 2 (0.98g) and dicyclohexylamine (0.44 ml) in DMF (10 ml). After 15min the mixture was diluted with pH 6 phosphate buffer (50 ml) and extracted with EA (3x50 ml). The combined extracts were washed with water (50 ml), dried and evaporated and the residue was purified

 

by chromatography eluting with 9:1 EA-methanol to give the <u>title compound</u> as an oil (0.8g).

Analysis Found: C, 70.55; H, 7.9; N, 3.1;

$C_{31}H_{41}NO_6$ requires: C, 71.1 ; H, 7.9; N, 2.7%

$[\alpha]_D^{25.5}$ = +61.0° ($CHCl_3$)

<u>Intermediate 11</u>

<u>[1R-[1α(Z),2β,5α]]-(-)-Methoxymethyl 7-[5-[[(1,1'-Biphenyl)-4-yl]methoxy]-2-(4-morpholinyl)-3-oxocyclopentyl]-4-heptenoate</u>

Pyridine-sulphur trioxide complex (0.64g) in DMSO (5 ml) was added to a cold (0°), stirred solution of Intermediate 10 (0.53g) and triethylamine (1.1ml) in $CH_2Cl_2$ (3ml). The mixture was allowed to attain ambient temperature over 3h and then poured into pH 6 phosphate buffer (50 ml) and extracted with EA (3x30 ml). The combined extracts were dried and evaporated and the residue was purified by chromatography using 17:3 EA-PE (b.p. 60-80°) to give the <u>title compound</u> as a solid (0.34g) m.p. 47.5-48.5°.

$[\alpha]_D^{26}$ = -7.45° ($CHCl_3$)

<u>Example 1</u>

<u>[(1α(Z),2β,5α]-(±)-7-[5-[[(1,1'-Biphenyl)-4-yl]methoxy]-2-(4-morpholinyl)-3-oxocyclopentyl]-4-heptenoic acid, compound with piperidine (1:1)</u>

<u>Method a)</u>

To a cooled (ice/water) stirred suspension of Intermediate 1 (60g) and triphenylmethyl chloride (35.4g) in $CH_2Cl_2$ (240ml) was added triethylamine (36.5ml). This mixture was stirred at less than 10° for 0.5h when more triethylamine (98ml) was added, followed by a solution of pyridine-sulphur trioxide (66.5g) in DMSO (240ml). The coolant was removed and the temperature of the mixture allowed to rise to 21° over 1.75h. Water (600ml) was added and the mixture extracted with ether (1x420ml, 1x300ml). The combined extracts were washed with water (600ml), 1M citric acid solution (480ml) and water (600ml), and then treated with trifluoroacetic acid (60ml)

at 20° for 20min. The solution was carefully neutralised with 8% $NaHCO_3$ solution (830ml) and the layers separated. The ethereal phase was washed with 20% brine (600ml), dried ($Na_2SO_4$), made up to a total volume of 1450ml (24vol) with more ether and stirred at 20° during the addition of piperidine (9.36g) in ether (50ml). The mixture was stored at 5° overnight and then filtered, washed with ether (3x180ml) and dried (3h/1mmHg, 21°) to give the title compound (40.9g), m.p. 101-104°.
T.L.C. $SiO_2$ $CH_2Cl_2$-MeOH-HOAc-$H_2O$ (95:4:0.5:0.5) Rf 0.31

Method b)

A solution of Intermediate 5 (0.512g) in ether (8ml) was stirred with trifluoroacetic acid (0.7ml) at 20° for 0.5h. Ether (25ml) was added and the mixture poured into 15% aqueous pH 6 phosphate buffer (30ml). The layers were separated and the ethereal phase washed with brine (20ml), dried and evaporated. The residue in ether (10ml) was treated with piperidine (0.068g) at 20° for 2h. The resultant solid was filtered off, washed with ether (3x4ml) and dried to give the title compound (0.328g), m.p. 103-106°.

Method c)

Trifluoroacetic acid (3ml) was added dropwise to a cooled (0°) stirred solution of Intermediate 7 (0.3g) in 1:1 $CH_2Cl_2$-ether (3ml). After 15 min the cooling bath was removed and the mixture stirred at ambient temperature for 2.25h. The mixture was diluted with pH 6 phosphate buffer (250ml) and extracted with ether (3x30ml). The combined extracts were washed with brine (20ml), dried and evaporated. A solution of the residue in ether (15ml) was treated with piperidine (0.046g) at 20° for 1.5h. The resultant solid was filtered off, washed with ether (2x10ml) and dried to give the title compound (0.11g) m.p. 95.5-99°.

Method d)

A mixture of Intermediate 9a (28mg) and activated zinc dust (ca. 0.5g) in THF (4ml) and pH 6.35 phosphate

buffer (0.8ml) was rapidly stirred at ambient temperature for 3h. The mixture was diluted with pH 6.35 buffer (20ml) and extracted with ether (3x20ml). The combined extracts were dried and evaporated and the residue was purified by chromatography using ether as eluent. The oil in ether (1ml) was treated with an excess of piperidine and stored at 4° for 19h. The precipitate was filtered off, dried and evaporated to give the <u>title compound</u> as a solid (9mg) m.p. 97.5-99°.

<u>Method e)</u>

A solution of Intermediate 9b (40mg) in trifluoroacetic acid (0.8ml) was kept at 20° for 10min then poured into pH 6.5 phosphate buffer (50ml) and extracted with EA (2x50ml). The combined extracts were washed with pH 6.5 phosphate buffer (2x40ml), dried and evaporated and the residue was purified by chromatography using ether as eluent. The residue in ether was treated with an excess of piperidine to give the <u>title compound</u> as a solid (4mg) m.p. 94-96.5°.

<u>Example 2</u>

<u>[1R-[1α(Z),2β,5α]]-(-)-7-[5-[[(1,1'-Biphenyl-4-yl]methoxy]-2-(4-morpholinyl)-3-oxocyclopentyl]-4-heptenoic acid, compound with piperidine (1:1)</u>

<u>Method a)</u>

A solution of Intermediate 6 (0.7g) in 1:1 $CH_2Cl_2$-ether (20ml) containing trifluoroacetic acid (0.75ml) was stirred at 10° for 1.5h. The mixture was diluted with pH 6 phosphate buffer (75ml) and extracted with ether (2x30ml). The combined extracts were washed with brine (20ml), dried and evaporated. The residue in ether (15ml) was treated with piperidine (100mg) at ambient temperature for 1h. The resultant solid was filtered off, washed with ether (2x10ml) and dried to give the <u>title compound</u> (0.36g) m.p. 97-101.5°

Analysis                Found: C,72.4; H,8.4; N,4.9;
$C_{29}H_{35}NO_5.C_5H_{11}N$ required: C,72.6; H,8.2; N,4.9%
$[\alpha]_D^{24}$ = -10.8° ($CHCl_3$)

Method b)

A solution of Intermediate 8 (50mg) in methanol (6ml) was treated with 0.5M $Na_2HPO_4$ (1.5ml) and stirred at 20° for 30h. The mixture was concentrated *in vacuo* at 20°, diluted with pH 6.5 phosphate buffer (30ml) and extracted with EA (4x20ml). The combined extracts were dried and evaporated and the residue was purified by chromatography using ether as eluent to give an oil. The oil in ether (0.5ml) was treated with piperidine (10mg) and cooled to 0° for 16h. The precipitate was filtered off, washed with ether (2x2ml) and dried to give the title compound (11mg) m.p. 101-102.5°.

Method c

Trifluoroacetic acid (3ml) was added dropwise to a cooled (0°) stirred solution of Intermediate 11a (0.5g) in 1:1 $CH_2Cl_2$-ether (3 ml). The cooling bath was removed and the mixture allowed to warm to ambient temperature over 1h. The mixture was neutralised with 8% $NaHCO_3$ solution, poured into pH 6 phosphate buffer (50ml) and extracted with ether (4x50ml). The combined extracts were dried and evaporated and the residue in ether (5 ml) was treated with piperidine (135mg). The precipitated solid was filtered off, washed with ether and dried to give the title compound (0.23g), m.p. 93-99°.

Example 3

[1α(Z),2β,5α]-(±)-7-[5-[[(1,1'-Biphenyl)-4-yl]methoxy]-2-(4-morpholinyl)-3-oxocyclopentyl]-4-heptenoic acid

A suspension of the product of Example 1 (2 g) in water (50 ml) and pH 6 phosphate buffer (100 ml) was extracted with ether (4 x 50 ml). The combined extracts were dried and evaporated to give a solid (1.69 g). A portion was crystallised from ether-isopentane to give the title compound m.p. 99-100°.

Claims:

1. A process for the preparation of a compound of formula (1)

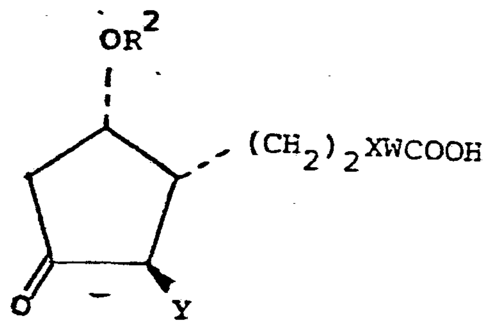

(1)

wherein X is cis or trans $-CH=CH-$; W is straight or branched $C_{1-7}$ alkylene; Y represents a saturated heterocyclic amino group which has 5-8 ring members and (a) optionally contains in the ring $-O-$, $-S-$, $-SO_2-$, $-NR^4$ (where $R^4$ is a hydrogen atom, $C_{1-7}$ alkyl or aralkyl having a $C_{1-4}$ alkyl portion); and/or (b) is optionally substituted by one or more $C_{1-4}$ alkyl groups;

$R^2$ is (i) $C_{3-6}$ alkenyl, optionally substituted by phenyl (the phenyl being optionally substituted by $C_{1-4}$ alkyl, $C_{1-4}$ alkoxy, halogen, $C_{5-7}$ cycloalkyl or phenyl ($C_{1-4}$) alkyl), biphenyl (optionally substituted by $C_{1-4}$ alkyl, $C_{1-4}$ alkoxy or halogen), or naphthyl; (ii) $C_{1-12}$ alkyl; (iii) $C_{1-5}$ alkyl substituted by (a) phenyl [optionally substituted by halogen, hydroxy, $C_{1-6}$ alkyl, $C_{1-6}$ alkoxy, $C_{1-4}$ hydroxyalkoxy, trifluoromethyl, cyano, aryloxy (e.g. phenoxy), $C_{5-7}$ cycloalkyl, aralkoxy (e.g. benzyloxy), dimethylaminomethyl, carboxamido ($-CONH_2$), thiocarboxamido ($-CSNH_2$), $C_{1-4}$ alkanoyl, $-NR^5R^6$ (where $R^5$ and $R^6$ are the same or different and are each a hydrogen atom or $C_{1-4}$ alkyl, or where $-NR^5R^6$ is a saturated heterocyclic amino group as defined above for Y), $C_{1-3}$ alkylthio, $C_{1-3}$ alkylsulphinyl, $C_{1-3}$ alkylsulphonyl, phenylalkyl having a $C_{1-3}$ alkyl portion, aminosulphonyl, $C_{1-3}$ alkanoylaminosulphonyl,

phenylsulphonyl (the phenyl portion being optionally substituted by $C_{1-3}$ alkyl or $C_{1-3}$ alkoxy), nitro, or thienyl], (b) thienyl or furanyl [the thienyl and furanyl groups being optionally substituted by $C_{1-6}$ alkyl, $C_{1-6}$ alkoxy, aryl (e.g. phenyl) or phenyl $(C_{1-3})$ alkyl or phenyl $(C_{1-3})$-alkoxy (the aryl or phenyl group in each case being optionally substituted by $C_{1-3}$ alkyl, $C_{1-3}$ alkoxy or halogen), aryloxy (e.g. phenoxy), $C_{5-7}$ cycloalkyl, halogen, nitro or thienyl], (c) biphenyl (optionally substituted by phenyl or one or two $C_{1-4}$ alkyl, $C_{1-4}$ alkoxy or halogen substituents), or (d) naphthyl (optionally substituted by $C_{1-4}$ alkyl, $C_{1-4}$ alkoxy or halogen);

and the physiologically acceptable salts and the solvates (e.g. hydrates) thereof, which process comprises hydrolysing an ester of formula (2)

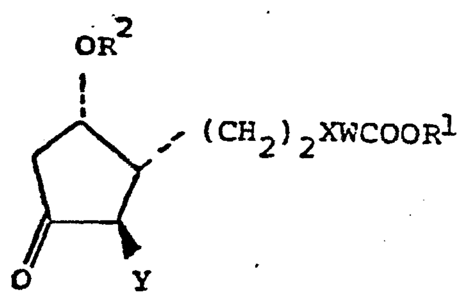

(2)

in which $R^1$ is:

(a) $-CR^7R^8R^9$ in which $R^7$ and $R^8$ are each phenyl (optionally substituted by $C_{1-4}$ alkyl, $C_{1-4}$ alkoxy, di-$(C_{1-4})$alkylamino, nitro or halogen) and $R^9$ is a hydrogen atom or a substituted or unsubstituted phenyl group as defined for $R^7$ and $R^8$;

(b) $-CH_2BR^{10}$ where B is -O- or -S-and $R^{10}$ is $C_{1-4}$ alkyl;

(c) $-CH_2OCOR^{11}$ where $R^{11}$ is $C_{1-4}$ alkyl or methoxy;

(d) $-CHOCOCH_3$ where $R^{12}$ is methyl or phenyl;
$\quad |$
$\quad R^{12}$

(e) tetrahydro-5-oxo-2-furanyl;

(f) $-CH_2CH_2SiR^{13}_3$ where $R^{13}$ is $C_{1-6}$ alkyl, e.g. methyl;

(g) $-CH_2CCl_3$; or

(h) $-SiR^{14}R^{15}R^{16}$ where $R^{14}$, $R^{15}$ and $R^{16}$ are aryl (e.g. phenyl) or $C_{1-6}$ alkyl, at least one of $R^{14}$, $R^{15}$ and $R^{16}$ being aryl,

and optionally thereafter (where the initial product is in the form of a salt) liberating the free acid from the salt and/or converting the salt into another salt, or (where the initial product is an acid) treating the acid with a base to form a salt.

2. A process as claimed in claim 1 in which $R^1$ is a group of type (a).

3. A process as claimed in claim 1 in which $R^1$ is triphenylmethyl.

4. A process as claimed in claim 2 or claim 3 which is effected in the presence of an organic acid.

5. A process as claimed in claim 2 or claim 3 which is effected in the presence of trifluoroacetic acid.

6. A process as claimed in any one of claims 1 to 5 which includes the step of preparing the compound of formula (2) by oxidising a corresponding hydroxy compound of formula (3)

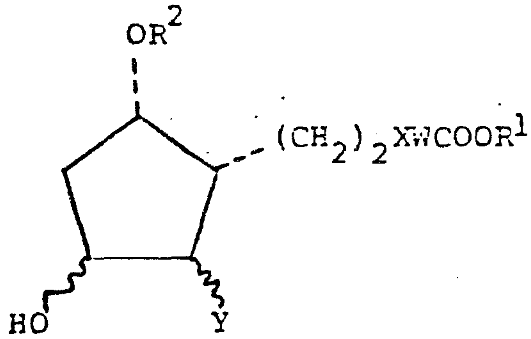

(3)

(other than a compound in which Y is in the α-position and the ring hydroxy group is in the β-position).

7. A process as claimed in any one of the preceding claims in which:

W is $C_{1-5}$ alkylene,

X is <u>cis</u> -CH=CH-,

Y is piperidino, morpholino, homomorpholino, thiomorpholino or 1,1-dioxothiomorpholino, and

$R^2$ is a phenylalkyl group in which the alkyl portion contains 1-3 carbon atoms and the phenyl is substituted with one of the following groups: $C_{1-3}$ alkylthio, thienyl or phenyl optionally substituted by $C_{1-3}$ alkyl, $C_{1-3}$ alkoxy, halogen or phenyl; or is thienylalkyl in which the alkyl portion contains 1-3 carbon atoms and the thienyl group is substituted by a phenyl group; or cinnamyl.

8. A process as claimed in any one of the preceding claims in which :

X is <u>cis</u> -CH=CH-,

W is $-CH_2CH_2-$

Y is morpholino or piperidino, and

$R^2$ is phenyl $(C_{1-3})$ alkyl in which the phenyl group is substituted by phenyl (which phenyl substituent is optionally substituted by $C_{1-3}$ alkyl, $C_{1-3}$ alkoxy or halogen); phenylthienylmethyl; or cinnamyl.

9. A process as claimed in any one of the preceding claims in which the compound produced is [1α(Z),2β,5α]-

(±)-7-[5-[[1,1'-biphenyl)-4-yl]methoxy]-2-(4-morpholinyl)-3-oxocyclopentyl]-4-heptenoic acid or a salt or hydrate thereof.

10. A process as claimed in any one of claims 1 to 8 in which the compound produced is [1R-[1α(Z),2β,5α]]-(-)-7-[5-[[(1,1'-biphenyl-4-yl]methoxy]-2-(4-morpholinyl)-3-oxocyclopentyl]-4-heptenoic acid or a salt or hydrate thereof.

European Patent Office

# EUROPEAN SEARCH REPORT

0078668

Application number

EP 82 30 5730

## DOCUMENTS CONSIDERED TO BE RELEVANT

| Category | Citation of document with indication, where appropriate, of relevant passages | Relevant to claim | CLASSIFICATION OF THE APPLICATION (Int. Cl. 3) |
|---|---|---|---|
| A | EP-A-0 032 432 (GLAXO GROUP LTD.) * Examples 2,6,8-11,13,19; claim 10d * | 1-10 | C 07 D 295/14<br>C 07 F 7/08 |
| A | GB-A-2 075 503 (GLAXO GROUP LTD.) * Claims * | 1-10 | |
| A | GB-A-2 028 805 (GLAXO GROUP LTD.) * Claims * | 1-10 | |

TECHNICAL FIELDS SEARCHED (Int. Cl. 3)

C 07 D 295/00

The present search report has been drawn up for all claims

| Place of search | Date of completion of the search | Examiner |
|---|---|---|
| THE HAGUE | 01-02-1983 | PAUWELS G.R.A. |

CATEGORY OF CITED DOCUMENTS

X : particularly relevant if taken alone
Y : particularly relevant if combined with another document of the same category
A : technological background
O : non-written disclosure
P : intermediate document
T : theory or principle underlying the invention
E : earlier patent document, but published on, or after the filing date
D : document cited in the application
L : document cited for other reasons
& : member of the same patent family, corresponding document